# EUROPÄISCHE PATENTSCHRIFT

(11) **EP 1 414 961 B1**
(45) Veröffentlichungstag und Bekanntmachung des Hinweises auf die Patenterteilung: **29.08.2007**
(21) Anmeldenummer: 02758317.8
(22) Anmeldetag: 05.07.2002
(51) Int. Cl.: C12N 15/11

(54) **NEUE OLIGORIBONUCLEOTID-DERIVATE ZUR GEZIELTEN HEMMUNG DER GENEXPRESSION**
NOVEL OLIGORIBONUCLEOTIDE DERIVATIVES FOR THE TARGETED INHIBITION OF GENE EXPRESSION
DERIVES D'OLIGORIBONUCLEOTIDE PERMETTANT L'INHIBITION ORIENTEE DE L'EXPRESSION GENIQUE

(30) Priorität: 12.07.2001 DE 10133915
(43) Veröffentlichungstag der Anmeldung: 06.05.2004
(73) Patentinhaber: Sanofi-Aventis Deutschland GmbH, 65929 Frankfurt am Main (DE)
(72) Erfinder: UHLMANN, Eugen, 61479 Glashütten (DE); HUBER, Jochen, 67133 Maxdorf (DE); GUNKEL, Niki, 69121 Heidelberg (DE); NEUMANN, Sandra, 63073 Offenbach (DE)
(86) Internationale Anmeldenummer: PCT/EP2002/007483
(87) Internationale Veröffentlichungsnummer: WO 2003/008595

(56) Entgegenhaltungen:
- WO-A-00/04189
- KANDIMALLA E R ET AL: "MIXED BACKBONE ANTISENSE OLIGONUCLEOTIDES: DESIGN, BIOCHEMICAL AND BIOLOGICAL PROPERTIES OF OLIGONUCLEOTIDES CONTAINING 2'-5'-RIBO- AND 3'-5'-DEOXYRIBONUCLEOTIDE SEGMENTS" NUCLEIC ACIDS RESEARCH, OXFORD UNIVERSITY PRESS, SURREY, GB, Bd. 25, Nr. 2, 1997, Seiten 370-378, XP001084034 ISSN: 0305-1048
- TORRENCE P F ET AL: "TARGETING RNA FOR DEGRADATION WITH A (2'-5')OLIGOADENYLATE-ANTISENSE CHIMERA" PROCEEDINGS OF THE NATIONAL ACADEMY OF SCIENCES OF USA, NATIONAL ACADEMY OF SCIENCE. WASHINGTON, US, Bd. 90, Nr. 4, 1. Februar 1993 (1993-02-01), Seiten 1300-1304, XP000644528 ISSN: 0027-8424 in der Anmeldung erwähnt
- TORRENCE P F ET AL: "OLIGONUCLEOTIDE STRUCTURAL PARAMETERS THAT INFLUENCE BINDING OF 5'-O-TRIPHOSPHOADENYLYL-(2' 5')-ADENYLYL-(2' 5')-ADENOSINE TO THE 5'-O-TRIPHOSPHOADENYLYL-(2' 5')-ADENYLY-(2' 5)-ADENOSINE DEPENDENT ENDORIBONUCLEASE: CHAIN LENGTH, PHOSPHORYLATION STATE, AN" JOURNAL OF MEDICINAL CHEMISTRY, AMERICAN CHEMICAL SOCIETY. WASHINGTON, US, Bd. 27, Nr. 6, 1984, Seiten 726-733, XP009003873 ISSN: 0022-2623
- LEAMAN DOUGLAS W ET AL: "Controlling gene expression with 2-5A antisense." METHODS (ORLANDO), Bd. 18, Nr. 3, Juli 1999 (1999-07), Seiten 252-265, XP002247621 ISSN: 1046-2023

### SEQUENZPROTOKOLL

<110> Aventis Pharma Deutschland GmbH
<120> Neue Oligoribonucleotid-Derivate zur gezielten Hemmung der Genexpression
<130> AVE D-2001/A034
<140>
   <141>
<160> 22
<170> PatentIn Ver. 2.1
<210> 1
   <211> 25
   <212> DNA
   <213> Künstliche Sequenz
<220>
   <223> Beschreibung der künstlichen Sequenz: Bereich aus Photinus pyralis Luziferase
<400> 1
   ttttgaagcg aaggttgtgg atctg 25
<210> 2
   <211> 25
   <212> RNA
   <213> Künstliche Sequenz
<220>
   <223> Beschreibung der künstlichen Sequenz: Bereich aus Photinus pyralis Luziferase
<400> 2
   uuuugaagcg aagguugugg aucug 25
<210> 3
   <211> 80
   <212> DNA
   <213> Künstliche Sequenz
<220>
   <223> Beschreibung der künstlichen Sequenz: Bereich aus Photinus pyralis Luziferase
<400> 3
   gcttttacag atgcacatat cgaggtggac atcacttacg cgaaaatgtc tacgtgtata 60
   gctccacctg tagtgaatgc                                             80
<210> 4
   <211> 80
   <212> DNA
   <213> Künstliche Sequenz
<220>
   <223> Beschreibung der künstlichen Sequenz: Bereich aus Photinus pyralis Luziferase
<400> 4
   ccgcgaacga catttataat gaacgtgaat tgctcaacag ggcgcttgct gtaaatatta 60
   cttgcactta acgagttgtc                                             80
<210> 5
   <211> 80
   <212> DNA
   <213> Künstliche Sequenz
<220>
   <223> Beschreibung der künstlichen Sequenz: Bereich aus Photinus pyralis Luziferase
<400> 5
   gcggtcggta aagttgttcc attttttgaa gcgaaggttg cgccagccat ttcaacaagg 60
   taaaaaactt cgcttccaac                                             80
<210> 6
   <211> 80
   <212> DNA
   <213> Künstliche Sequenz
<220>
   <223> Beschreibung der künstlichen Sequenz: Bereich aus Photinus pyralis Luziferase
<400> 6
   attttttgaa gcgaaggttg tggatctgga taccgggaaa taaaaaactt cgcttccaac 60
   acctagacct atggcccttt                                             80
<210> 7
   <211> 40
   <212> RNA
   <213> Künstliche Sequenz
<220>
   <223> Beschreibung der künstlichen Sequenz: Bereich aus Photinus pyralis Luziferase
<400> 7
   gcuuuuacag augcacauau cgagguggac aucacuuacg 40
<210> 8
   <211> 40
   <212> RNA
   <213> Künstliche Sequenz
<220>
   <223> Beschreibung der künstlichen Sequenz: Bereich aus Photinus pyralis Luziferase
<400> 8
   ccgcgaacga cauuuauaau gaacgugaau ugcucaacag 40
<210> 9
   <211> 40
   <212> RNA
   <213> Künstliche Sequenz
<220>
   <223> Beschreibung der künstlichen Sequenz: Bereich aus Photinus pyralis Luziferase
<400> 9
   gcggucggua aaguuguucc auuuuuugaa gcgaagguug 40
<210> 10
   <211> 40
   <212> RNA
   <213> Künstliche Sequenz
<220>
   <223> Beschreibung der künstlichen Sequenz: Bereich aus Photinus pyralis Luziferase
<400> 10
   auuuuuugaa gcgaagguug uggaucugga uaccgggaaa 40
<210> 11
   <211> 26
   <212> RNA
   <213> Künstliche Sequenz
<220>
   <223> Beschreibung der künstlichen Sequenz: Bereich aus Photinus pyralis Luziferase
<400> 11
   caccucgaua ugugcaucug uaaaaa 26
<210> 12
   <211> 25
   <212> RNA
   <213> Künstliche Sequenz
<220>
   <223> Beschreibung der künstlichen Sequenz: Bereich aus Photinus pyralis Luziferase
<400> 12
   gagcaauuca cguucauuau aaaaa 25
<210> 13
   <211> 25
   <212> RNA
   <213> Künstliche Sequenz
<220>
   <223> Beschreibung der künstlichen Sequenz: Bereich aus Photinus pyralis Luziferase
<400> 13
   cagauccaca accuucgcuu caaaa 25
<210> 14
   <211> 25
   <212> RNA
   <213> Künstliche Sequenz
<220>
   <223> Beschreibung der künstlichen Sequenz: Bereich aus Photinus pyralis Luziferase
<400> 14
   cagauccaca accuucgcuu caaaa 25
<210> 15
   <211> 25
   <212> RNA
   <213> Künstliche Sequenz
<220>
   <223> Beschreibung der künstlichen Sequenz: Bereich aus humaner EDG1
<400> 15
   cagagccacc aacuucucuu caaaa 25
<210> 16
   <211> 60
   <212> RNA
   <213> Künstliche Sequenz
<220>
   <223> Beschreibung der künstlichen Sequenz: Bereich aus humaner EDG1
<400> 16
   gaccucggug guguucauuc ucaucugcug cuuuaucauc cuggagaaca ucuuugucuu 60
<210> 17
   <211> 60
   <212> RNA
   <213> Künstliche Sequenz
<220>
   <223> Beschreibung der künstlichen Sequenz: Bereich aus humaner EDG1
<400> 17
   auuuccaagg ccagccgcag cucugagaau guggcgcugc ucaagaccgu aauuaucguc 60
   <210> 18
   <211> 25
   <212> RNA
   <213> Künstliche Sequenz
<220>
   <223> Beschreibung der künstlichen Sequenz: Bereich aus humaner EDG1
<400> 18
   aaagauguuc uccaggauga uaaaa 25
<210> 19
   <211> 25
   <212> RNA
   <213> Künstliche Sequenz
<220>
   <223> Beschreibung der künstlichen Sequenz: Bereich aus humaner EDG1
<400> 19
   cagagcugcg gcuggccuug gaaaa 25
<210> 20
   <211> 25
   <212> RNA
   <213> Künstliche Sequenz
<220>
   <223> Beschreibung der künstlichen Sequenz: Bereich aus humaner EDG1
<400> 20
   cacagcggcg ucuguccgug gaaaa 25
<210> 21
   <211> 21
   <212> RNA
   <213> Künstliche Sequenz
<220>
   <223> Beschreibung der künstlichen Sequenz: Bereich aus Photinus pyralis Luziferase
<400> 21
   gaagcgaagg uuguggaucu g 21
<210> 22
   <211> 16
   <212> RNA
   <213> Künstliche Sequenz
<220>
   <223> Beschreibung der künstlichen Sequenz: Bereich aus Photinus pyralis Luziferase
<400> 22
   accagaccac aacccg 16

## Beschreibung

Die vorliegende Erfindung bezieht sich auf neue Oligoribonucleotid-Derivate, die am 3'-Ende einen 2'5'-verknüpften Oligoribonucleotid-Rest ohne 5'-Phosphat-Rest aufweisen und deren Verwendung zur gezielten Inhibition der Genexpression.

Die Hemmung der Genexpression mit Hilfe von synthetischen Nucleinsäuren gewinnt zunehmende Bedeutung. Typische Vertreter dieser synthetischen Nucleinsäuren (Oligonucleotide) sind Antisense Oligonucleotide, Ribozyme, DNA-Enzyme und External Guide Sequences (EGS). "Antisense Oligonucleotide" sind kurze einzelsträngige Nucleinsäure-Derivate, die über Watson-Crick Basenpaarung an eine komplementäre Botenribonucleinsäure (mRNA) binden, deren Übersetzung in das entsprechende Protein gehemmt werden soll. In den meisten Fällen entfalten Antisense Oligonucleotide ihre Wirkung nach einem Mechanismus, der von der zellulären Ribonuclease H (RNase H) unterstützt wird, was durch zahlreiche Studien belegt ist. Die in allen Zellen vorkommende RNase H erkennt einen Doppelstrang aus DNA und RNA und schneidet die zum Oligonucleotid komplementäre mRNA durch Hydrolyse einer oder meist mehrerer Phosphodiester-Bindungen. Bekannt ist, wie die Oligonucleotide modifiziert sein müssen, damit eine Aktivierung der RNase H stattfinden kann. Dies ist beispielsweise in "Uhlmann (2000) Curr. Opin. Drug Discov. Dev. 3, 203-213" beschrieben. Synthetische Ribozyme bringen diese Nucleaseaktivität in Ihrer Sequenz mit. Der bekannteste Ribozymtyp sind die sogenannten "Hammerhead" Ribozyme, in welchen die von natürlich vorkommenden Ribozymen abgeleitete Konsensus-Sequenz GAAAC den RNase-Teil bildet und die flankierenden Sequenzen den Antisense Oligonucleotid-Teil bilden. In ähnlicher Weise wirken DNA-Enzyme, die sich allerdings nicht von natürlich vorkommenden Ribozym-Motiven ableiten, sondern durch in vitro Selektion gefunden wurden. EGS sind synthetische RNA-Analoga, welche die zelluläre RNase P aktivieren und über entsprechende flankierende Sequenzen an die Ziel-mRNA binden und einen spezifischen mRNA-Abbau einleiten.

Ein gemeinsames Problem der Inhibition der Genexpression mit Hilfe synthetischer Oligonucleotide besteht darin, dass stets eine größere Anzahl von Oligonucleotiden gegen verschiedene Bereiche der Ziel-Nucleinsäure getestet werden müssen, um eine effiziente Sequenz zu identifizieren. Weiterhin erfolgt die Inhibition der Genexpression durch Antisense Oligonucleotide oft nur ineffizient oder unvollständig. Zudem wurden sequenz-unspezifische Nebenwirkungen beobachtet, die darauf beruhen können, dass relativ kurze Teilsequenzen von etwa fünf Basen Länge bereits die RNase H aktivieren. Dies wird beispielsweise von "Woolf et al. (1992). Proc. Natl. Acad. Sci. U. S. A. 89, 7305-7309)" gezeigt. Es gibt aber auch Nebeneffekte, die auf der Wechselwirkung der Antisense Oligonucleotide mit Proteinen beruhen.

Kürzlich wurde die Verwendung doppelsträngiger RNA zur Hemmung der Genexpression beschrieben. Doppelsträngige RNA (dsRNA) ist für bestimmte Zellen und Organismen ein Signal, einen sequenzspezifischen Abbau der mRNA nach einem Prozess einzuleiten, der als RNA-Interferenz (RNAi) bekannt ist. Das Phänomen des RNAi wurde in einer Reihe verschiedener Organismen beobachtet, wie beispielsweise C. elegans, Fliegen, Pilzen, Pflanzen und Maus-Embryonen. Man glaubt, dass RNAi dem in Pflanzen beobachten Post-Transcriptional Gene Silencing (PTGS) sehr ähnlich bzw. mit diesem identisch ist. Durch einfache Injektion von dsRNA mit einer Länge von mehr als 500 Basenpaaren (bp), deren Sense-Strang Sequenz mit der zu inhibierenden Ziel-mRNA identisch ist, kann die Expression eines Ziel-Gens mit der entsprechenden DNA-Sequenz spezifisch gehemmt werden. Die Genexpression nicht homologer Gene wird dabei nicht beeinträchtigt. Die Basensequenz des Ziel-Gens wird dabei nicht verändert. RNAi ist ein posttranskriptioneller Prozess, bei dem die dsRNA zuerst in kleinere Fragmente gespalten wird, welche dann wahrscheinlich zum sequenzspezifischen Abbau der Ziel-mRNA Verwendung finden.

Bisher wurde eine effiziente Inhibition der Genexpression hauptsächlich mit dsRNA von über 100 bp Länge erreicht. Diese relativ lange dsRNA ist nur über in vitro oder in vivo Transkription aus der entsprechenden DNA über geeignete Transkriptionssysteme zugänglich ist. Eine andere Limitierung des RNAi mit langer dsRNA besteht darin, dass nur bestimmte Organismen, wie C. elegans, Zebrafisch, Pflanzen, bestimmte Pilzsorten, Drosophila, Oocyten und Embryonen von Mäusen, sequenzspezifische Inhibition mit dsRNA zulassen, während die meisten tierischen Zellen bei Behandlung mit dsRNA Apoptose auslösen. Bei langer dsRNA wird auch noch eine Inhibition der Genexpression beobachtet, wenn die Sequenzhomologie 70 bis 90% beträgt. Daher kann es bei Gen-Familien mit hoher Sequenzhomologie zu Fehlinterpretationen des Phänotyps kommen, indem die Expression mehrerer nicht vollständig homologer Gene gleichzeitig gehemmt wird.

Die Behandlung von Zellen mit dsRNA, wie z. B. mit dsRNA-Viren, führt im allgemeinen zu einem apoptotischen Prozess bzw. zum sequenzunspezifischen Abbau der mRNA infolge der Induktion einer 2'5'-Oligoadenylat-Synthase-Aktivität. Als Antwort auf die virale dsRNA synthetisiert die infizierte Zelle trimeres oder tetrameres Adenylat (2'5'-A) mit der ungewöhnlichen 2'5'-Phosphodiester Internucleosidbrücke. Das 2'5'-A wird durch zelluläre Kinasen am 5'-Ende phosphoryliert und aktiviert dann eine Nuclease, die RNase L genannt wird. Man kann das 2'5'-A auch chemisch synthetisieren und der Zelle zuführen (Torrence et al. (1994) Curr. Med. Chem 1,176-191). Das synthetische 2'5'-A aktiviert die RNase L aber nur, wenn es in das 5'-Phosphat oder 5'-Triphosphat übergeführt wurde. Die durch 5'-p-2'5'-A (p bedeutet Phosphat, Diphosphat oder Triphosphat) aktivierte RNase L baut dann die gesamte RNA der Zelle in sequenzunspezifischer Weise ab. Es wurde auch gezeigt, dass man mit Hilfe von Antisense Oligonucleotid-Konjugaten mit einem 5'-p-2'5'-A-Rest die Genexpression sequenzspezifisch hemmen kann. Dazu ist es aber essentiell, dass das 5'-Ende des 2'5'-A Restes nicht mit dem Oligonucleotid verknüpft ist, sondern als Phosphat, Thiophosphat oder Triphosphat vorliegt (Torrence et al. (1993) Proc. Natl. Acad. Sci. U. S. A. 90, 1300-4). Weiterhin muss der die Ziel-RNA erkennende Oligonucleotidteil (Antisense-Teil) in einzelsträngiger Form vorliegen. Oligonucleotide mit 2'5'-A Resten an den 3'-Enden, die folglich keine freie 5'-Phosphat- oder Triphosphat-Funktion aufweisen, sind aus oben genannten Gründen bisher nicht zur Inhibition der Genexpression beschrieben worden. Die Hemmung der Genexpression mit den einzelsträngigen 5'-phosphorylierten 5'-p-2'5'-A-Antisense Oligonucleotid-Konjugaten ist eine Variation des Antisense Prinzips und unterliegt somit auch den Limitierungen des Antisense Oligonucleotid-Ansatzes. Der 2'5'-A-Rest wird dabei über einen Spacer (Linker) an das 5'-Ende des Oligonucleotids angehängt, so dass das 2'- bzw. 3'-Ende des 2'5'-A-Restes gebunden vorliegt. Der RNA-bindende Teil besteht bevorzugt aus DNA (Abbildungen 4 bis 6 in Torence, Curr. Opin. Mol. Ther. (1999) 1, 307).

In letzter Zeit werden Oligonucleotide in zunehmenden Maße als Werkzeuge zum Studium der Funktion neuer Gene verwendet (Functional Genomics). Der Einsatz von Antisense Oligonucleotiden und Ribozymen zur sequenzspezifischen Inhibition der Genexpression neuer Gene, die Proteine mit unbekannter Funktion kodieren, wird dadurch erschwert, dass im allgemeinen viele verschiedene Oligonucleotide unterschiedlicher Sequenzen getestet werden müssen, was besonders im großen Durchsatz hinderlich ist.

So offenbart US 5,886,165 Oligonukleotide aus Deoxyribonukleotiden mit 3'-5'-Verknüpfungen und Ribonukleotiden mit 2'-3'-Verknüpfungen. WO 00/04189 offenbart modifizierte Oligonukleotide mit wenigstens einer 2',5'-Verknüpfung.

Die Aufgabe dieser Erfindung bestand also darin, neue chemisch modifizierte Oligonucleotide mit signifikant verbesserter Hemmung der Genexpression zur Verfügung zu stellen, welche die oben genannten Limitierungen der herkömmlichen Methoden und Agenzien umgehen. Insbesondere sollte die Genexpression in einem RNA-Interferenz-ähnlichen Prozess gehemmt werden.

Die Aufgabe wurde erfindungsgemäß gelöst durch neue Oligonucleotid-Derivate, die am 3'-Ende einen 2'5'-verknüpften Oligonucleotid-Rest aufweisen, wobei dieser keine freie Phosphat-, Thiophosphat- oder Triphosphatgruppe trägt. Die Sequenz der neuen Oligonucleotid-Derivate ist komplementär zur RNA-Sequenz, deren Translation gehemmt werden soll.

Die Erfindung betrifft daher Oligonucleotid-Derivate der Formel I,

5'-(N)ₓ-(Z)ₙ Formel I

wobei
N natürlich oder nicht natürlich vorkommende Ridonucleotide sind, die zumindest teilweise zu einer Ziel-RNA komplementär sind,
x unabhängig gleich 10 bis 100, bevorzugt 15 bis 45, und besonders bevorzugt 16 bis 25 ist,
n gleich 2 bis 20, bevorzugt 3 bis 10, besonders bevorzugt 3 bis 6 ist,
Z natürlich oder nicht natürlich vorkommende Nucleotide sind, die mit einer 2'5'-Internucleosid-Bindung verknüpft sind,
mit der Maßgabe, dass deren homologe Ziel-RNA folgende Sequenzmuster aufweisen:
5'-(U)ᵥ-(N')_{z}-(U)_{w}
5'-(U)ᵥ-(N')_{z}-UX
5'-UX-(N')_{z}-UX und
5'-(U)ᵥ-(N')_{z}
wobei v und w unabhängig voneinander gleich 2 bis 20, bevorzugt 2 bis 10, besonders bevorzugt 3 bis 6 sind und z gleich 15 bis 25, bevorzugt 16 bis 23 und besonders bevorzugt 19 bis. 21 ist und U gleich Uridin, N gleich Adenosin (A), Guanosin (G), Cytidin (C) oder U, und X gleich A, G oder C, bevorzugt A, sind. N kann in einer bevorzugten Ausführungsform ein Ribonukleotid sein.

Enthält das Gen, dessen Expression gehemmt werden soll, beispielsweise folgende DNA-Sequenz
5'-TTTTGAAGCGAAGGTTGTGGATCTG (Seq ID Nr. 1)
bzw. folgende RNA-Sequenz
5'-UUUUGAAGCGAAGGUUGUGGAUCUG (Seq ID Nr. 2)
dann hat die Ziel-RNA folgendes Sequenzmuster
5'-(U)ᵥ-(N)_{z}-UX, wobei v gleich 4, z gleich 19 und X gleich G sind.

Weiterhin bevorzugt sind Oligonucleotide der Formel I, in denen eine oder mehrere Phosphodiester-Bindungen ersetzt sind, beispielsweise durch Phosphorothioat-Bindungen oder N3',P5'-Phosphoramidat-Bindungen . Besonders bevorzugt sind Oligonucleotide der Formel I, in denen eine oder mehrere Phosphodiester-Bindungen durch Phosphorothioat-Reste ersetzt sind. Die Einführung der Phosphorothioat-Reste erfolgt vorzugsweise an den 3'-Enden, den 5'-Enden sowie an den internen Pyrimidin-Nucleotiden C und U, insbesondere dann, wenn mehrere Pyrimidin-Nucleotide in der Sequenz aufeinander folgen.

Eine besondere Ausführungsform der Erfindung besteht darin, dass zwei oder mehrere Oligonucleotid-Derivate gemäss Fomel I als Gemisch zur Inhibition der Genexpression eingesetzt werden. Dabei können die Oligonucleotid-Derivate gegenunterschiedliche Regionen einer RNA oder gegen die RNA unterschiedlicher Gene gerichtet sein.
Die einzelsträngigen Oligonucleotide der Formel I wurden ursprünglich als Kontroll Oligonucleotide für RNAi Versuche mit kurzer dsRNA eingesetzt. Aufgrund des einzelsträngigen Charakters war also nicht mit einer Inhibition der Genexpression zu rechnen. Überraschenderweise hemmten bestimmte einzelsträngige Oligonucleotide die Expression aber auch, insbesondere wenn diese eine ausreichende Stabilität gegenüber Nucleasen aufwiesen. Überraschend war auch, dass die Oligonucleotide der Formel I, in denen der 2'5'-verknüpfte Oligoadenylat-Rest kein freies 5'-Phosphat-, 5'-Thiophosphat- oder 5'-Triphosphat-Rest aufweist, die Genexpression in sequenzspezifischer Weise zu hemmten. Es war auch völlig überraschend, dass dabei der 2'5'-verknüpfte Oligoadenylat-Rest über die 5'-Funktion direkt an die 3'5'-verknüpfte RNA gebunden sein kann. Bisher galt das Dogma, dass der 2'5'-verknüpfte Oligoadenylat-Rest am 5'-Ende einen freien Phosphat-, Thiophosphat oder Triphosphat-Rest tragen muss, damit die Genexpression gehemmt wird. Außerdem hatte man bisher eine 2'5'-oligoadenylt-vermittelte Inhibition stets mit einem unspezifischen, also sequenz-unabhängigen, Effekt in Zusammenhang gebracht (Bass, Nature (2001) 411, 428). Es ist daher offensichtlich, dass die Oligonucleotide der Formel I nicht nur in ihrer Struktur von den von Torrence (Curr. Opin. Mol. Ther. (1999) 1, 307) beschriebenen Oligonucleotid-Konjugaten abweicht, sondern darüber hinaus eine viel bessere Hemmwirkung zeigen, die folglich auf einem anderen Wirkmechanismus beruht.

Überraschenderweise waren die erfindungsgemässen Oligonucleotide auch in primären humanen Zellen in sequenzspezifischer Weise inhibitorisch wirksam. Unseres Wissens wurde die Inhibition der Genexpression durch Oligonucleotide mit 2'5'-verknüpften Nucleotiden bislang noch nicht in primären menschlichen Zellen beobachtet.

Die erfindungsgemässen Oligonucleotide der Formel I können auch zur Hemmung der Genexpression in Zellen, die nur wenig, keine oder defekte 2'5'-Oligoadanylat-Synthase exprimieren, eingesetzt werden.

Weiterhin können die Oligonucleotide der Formel I auch zur Behandlung von Patienten mit einer Defizienz oder einem Defekt der 2'5'-Oligoadenylat-Synthase eingesetzt werden. Beispielsweise können auch Patienten mit CFS (Chronic Fatigue Syndrome) behandelt werden.

Die Auswahl der Sequenzen der Oligonucleotide der Formel I, die zur Inhibition der Genexpression bestimmter Targets eingesetzt werden, erfolgt anhand der entsprechenden Gen-Sequenzen. Die Sequenzen dieser Gene werden durch Sequenzierung oder aus Gen-Datenbanken erhalten. Als Beispiel sei hier die Inhibition der Luciferase (firefly) mit doppelsträngigen Nucleinsäuren erläutert. Die Zugangsnummer für dieses Gen ist U47298. Der codierende Bereich der Firefly Luciferase umfasst 1653 Nucleotide. Es können beispielsweise unter anderem die folgenden vier Bereiche als Zielsequenzen für die Inhibition mit doppelsträngigen Nucleinsäuren ausgewählt werden.

Die entsprechende RNA für diese Bereiche hat dann folgende Sequenz.
GCUUUUACAGAUGCACAUAUCGAGGUGGACAUCACUUACG
   (Seq ID Nr. 7)
CCGCGAACGACAUUUAUAAUGAACGUGAAUUGCUCAACAG
   (Seq ID Nr. 8
GCGGUCGGUAAAGUUGUUCCAUUUUUUGAAGCGAAGGUUG
   (Seq ID Nr. 9
AUUUUUUGAAGCGAAGGUUGUGGAUCUGGAUACCGGGAAA
   (Seq ID Nr. 10

Die davon abgeleiteten erfindungsgemäßen komplementären Oligonucleotide der Formel I haben folgende Sequenzen und sind dadurch charakterisiert, dass zwei oder mehr Nucleotide (hier mit kleinen Buchstaben gekennzeichnet) durch eine 2'5'-Internucleosid-Bindung verknüpft sind. Bevorzugt sind 2'5'-verknüpfte Adenylat-Reste.
3' aaaaAUGUCUACGUGUAUAGCUCCAC Seq ID Nr. 11
3' aaaaAUAUUACUUGCACUUAACGAG Seq ID Nr. 12
3' aaaaCCAUUUCAACAAGGUAAAAAA Seq ID Nr. 13
3' aaaaaaCUUCGCUUCCAACACCUAGAC Seq ID Nr. 14

Die Oligonucleotide können zur Verbesserung der metabolischen Stabilität auch modifiziert werden, beispielsweise als Phosphorothioat (Sternchen). Die Stabilisierung durch Phosphorothioat erfolgt bevorzugt an den Enden und internen Pyrimidin-Nucleotiden.
3' a*a*a a-C*U*U*C G C*U*U C*C A A*C A C*C*U A G A*C

Die Spezifität der Inhibition der Luciferase-Expression kann anhand von Kontroll-Oligonucleotiden überprüft werden, die nicht vollständig komplementär zur Ziel-RNA sind und beispielsweise 4 Basen-Mißpaarungen aufweisen.
3' a*a*a a C*U*U*C U*C U*U*C A A C*C A*C*C G A*G A*C
   Seq ID Nr. 15

Oligonucleotide der Formel I haben beispielsweise folgende Struktur: 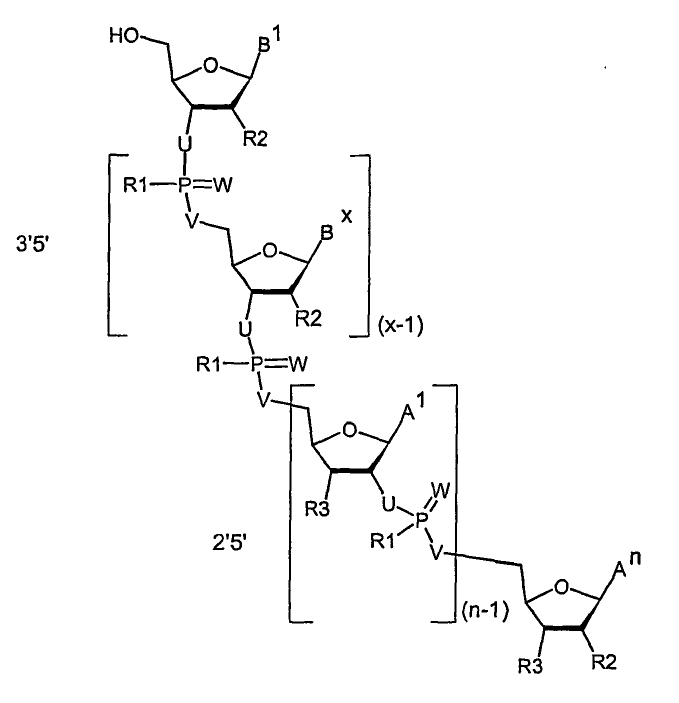 wobei B eine natürlich oder nicht natürlich vorkommende Nucleobase ist,
U, V und W unabhängig voneinander O, S, NH oder CH₂ sind, bevorzugt O oder S,
R¹ unabhängig voneinander OH, SH, CH₃ oder BH₃, bevorzugt OH oder SH ist, bzw. deren physiologisch verträglichen Salze
R² unabhängig voneinander OH, H, O-C₁ bis C₁₂-Alkyl , bevorzugt OH (Ribonucleotid) ist, wobei C₁ bis C₁₂-Alkyl bevorzugt CH₃ oder CH₃-O-CH₂CH₂ ist,
R³ unabhängig voneinander OH, H, O-C₁ bis C₁₂-Alkyl , bevorzugt OH oder H ist, wobei C₁ bis C₁₂-Alkyl bevorzugt CH₃ oder CH₃-O-CH₂CH₂ ist,
x unabhängig gleich 10 bis 100, bevorzugt 15 bis 45, und besonders bevorzugt 16 bis 25 ist,
n gleich 2 bis 20, bevorzugt 3 bis 10, besonders bevorzugt 3 bis 6 ist,

A für Adenin oder ein Adenin-Derivat, beispielsweise 8-Bromadenin, 8-Methyladenin, oder Hypoxanthin steht.

Zur Testung der Inhibition der Genexpression mit Hilfe der erfindungsgemässen Oligonucleotide in tierischen Zellen, insbesondere in primären menschlichen Zellen werden diese beispielsweise gegen ein menschliches Gen bzw. die zugehörige RNA gerichtet und in menschlichen Zellen (HUVEC, human umbilical vein endothelial cells) getestet. Hierzu kann beispielsweise die Edg-1 DNA (Zugangsnummer M31210) aus der Gendatenbank in die entsprechende Boten-RNA umgeschrieben und folgende zwei Bereiche (175 und 725) zur Synthese entsprechender Oligonucleotide ausgewählt werden.

Edg-1 RNA:
"175"
GACCUCGGUGGUGUUCAUUCUCAUCUGCUGCUUUAUCAUCCUGGAGAACAUCUUUGUCUU
   (Seq ID Nr. 16)
"725"
AUUUCCAAGGCCAGCCGCAGCUCUGAGAAUGUGGCGCUGCUCAAGACCGUAAUUAUCGUC
   (Seq ID Nr. 17)

Die entsprechenden Oligonucleotide hätten beispielsweise einen wie folgt offenbarten Aufbau:
3' -aaaaUAGUAGGACCUCUUGUAGAAA Seq ID Nr. 18;
3' -aaaaGGUUCCGGUCGGCGUCGAGAC Seq ID Nr. 19;
Mismatch Kontrolle
3'-aaaaGGUGCCUGUCUGCGGCGACAC Seq ID Nr. 20;

Die Mismatch Kontrolle stimmt in 5 Nucleotiden (als mismatch unterstrichen) nicht mit der edg-1 RNA überein.

Weiterhin wurden folgende gegen edg-1 gerichtete Oligonucleotide mit verbesserter Nucleasestabilität und erhöhter inhibitorischer Aktivität hergestellt, die sich von den vorherigen edg-1 Sequenzen ableiten.
3'-a*a*a a U*A G*U A G G A C*C*U C*U*U G*U*A G A A*A
3'-a*a*a a G G U*U*C*C G G*U*C G G*C G*U*C G A G A*C
3'-a*a*a a G G U*G C*C*U G*U*C*U G*C G G*C G A*C A*C

Die erfindungsgemässen Nucleinsäure-Derivate der Formel I sind aus Oligonucleotiden aufgebaut. Ein Oligonucleotid kann beispielsweise vollständig aus den Nukleotiden Adenosinphosphat, Guanosinphosphat, Inosinphosphat, Cytidinphosphat, Uridinphosphat und Thymidinphosphat aufgebaut sein. Bevorzugt sind Oligonucleotide, die aus Ribonucleotiden aufgebaut sind, die sogenannten Oligoribonucleotide. In anderen Ausführungsformen der Erfindung kann ein Oligonucleotid gegebenenfalls eine oder mehrere Modifikationen, beispielsweise chemische Modifikationen, enthalten. Ein Oligonucleotid kann mehrere gleiche und/oder verschiedene Modifikationen aufweisen.

Der 2'5'-verknüpfte Rest kann beispielsweise Adenosin, 3'-Deoxyadenosin (Cordycepin), Inosin, 8-Bromadenosin, 8-Methyladenosin und andere 8-substituierte Adenosin-Derivaten enthalten. Der Ribose-Rest kann auch als 3'-O-Methyladenosin derivatisiert sein. Die Internucleosidbindungen im 2'5'-verknüpften Teil sind bevorzugt Phosphodiester- und Phosphorothioatbindungen. Gängige Derivate des 2'5'-Adenylats, deren Synthese und Aktivierung der RNase L, sind in der Lituratur beschrieben (Player et al. (1998) Pharmacol. Ther. 78, 55).

Beispiele für chemische Modifikationen sind dem Fachmann bekannt und beispielsweise in E. Uhlmann and A. Peyman, Chemical Reviews 90 (1990) 543 und "Protocols for Oligonucleotides and Analogs" Synthesis and Properties & Synthesis and Analytical Techniques, S. Agrawal, Ed, Humana Press, Totowa, USA 1993, J. Hunziker und C. Leumann 'Nucleic Acid Analogs: Sythesis and Properties' in Modern Synthetic Methods (Ed. Beat Ernst und C. Leumann) Verlag Helvetica Chimica Acata, Basel, S 331-417, RP Iyer et al. Curr Opin Mol Therap (1999) 1:344-358; S. Verma und F. Eckstein , Annu Rev Biochem (1998) 67:99-134; JW Engels und E. Uhlmann : Chemistry of oligonucleotides. In: Pharmaceutical aspects of oligonucleotides. Couvreur P, Malvy C (Eds), Taylor & Francis, London, (2000): 35-78, beschrieben.

Die chemische Modifikation eines Oligonucleotids kann beispielsweise
a) den vollständigen oder teilweisen Ersatz der Phosphorsäurediesterbrücken, beispielsweise durch Phosphorothioat-, Phoshorodithioat-, NR¹R^{1'-}Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, (C₁-C₈)Alkylphosphonat- und/oder (C₆-C₁₂)-Arylphosphonat-Brücken,
   wobei
   R¹ und R^{1'} unabhängig voneinander für Wasserstoff, (C₁-C₁₈)-Alkyl, (C₆-C₂₀)-Aryl, (C₆-C₁₄)-Aryl-(C₁-C₈)-alkyl, bevorzugt für Wasserstoff, (C₁-C₈)-Alkyl und/oder Methoxyethyl, besonders bevorzugt für Wasserstoff, (C₁-C₄)-Alkyl und/oder
   Methoxyethyl stehen
   oder
   R¹ und R^{1'} zusammen mit dem sie tragenden Stickstoffatom einen 5-6-gliedrigen heterocyclischen Ring bilden, der zusätzlich ein weiteres Heteroatom aus der Reihe O, S, N enthalten kann;
b) den vollständigen oder teilweisen Ersatz der 3'- und/oder 5'-Phosphorsäurediesterbrücken durch "Dephospho"-Brücken (beschrieben beispielsweise in Uhlmann, E. und Peyman, A. in "Methods in Molecular Biology", Vol. 20, "Protocols for Oligonucleotides and Analogs", S. Agrawal, Ed., Humana Press, Totowa 1993, Chapter 16, 355ff), beispielsweise durch Formacetal, 3'-Thioformacetal, Methylhydroxylamin, Oxim, Methylendimethylhydrazo, Dimethylensulfon und/oder Silylgruppen;
c) den teilweisen Ersatz des Zuckerphosphat-Rückgrats, beispielsweise durch "Morpholino"-Oligomere (beispielweise in E. P. Stirchak et al., Nucleic Acids Res. 17 (1989) 6129 und in J. Summerton and D. Weller, Antisense and Nucleic Acid Drug Dev. 7 (1997) 187-195 beschrieben) und/oder durch Polyamid Nucleinsäuren ("PNAs") (beispielsweise beschrieben in P. E. Nielsen et al, Bioconj. Chem. 5 (1994) 3) und/oder Phosphomonosäureester Nukleinsäuren ("PHONAs") (beschrieben beispielsweise in Peyman et al., Angew. Chem. Int. Ed. Engl. 35 (1996) 2632-2638);
d) den teilweisen Ersatz der β-D-Riboseeinheiten, beispielsweise durch β-D-2'-Desoxyribose, α-D-2'-Desoxyribose, L-2'-Desoxyribose, 2'-F-2'-Desoxyribose, 2'-F-2'-Desoxyarabinofuranose, 2'-O-(C₁-C₆)Alkyl-Ribose, 2'-O-(C₂-C₆)Alkenyl-Ribose,2'-[O-(C₁-C₆)Alkyl-O-(C₁-C₆)Alkyl]-Ribose, 2'-NH₂-2'-desoxyribose, β-D-Xylofuranose, β-D-Arabinofuranose, α-Arabinofuranose, 2,4-Dideoxy-β-D-erythro-hexo-pyranose, konformativ eingeschränkte Zuckeranaloga wie LNA (Locked nucleic acids; Singh et al., Chem. Commun. 4 (1998) 455; Singh et al. Chem. Commun. 12 (1998) 1247) und carbocyclische (beschreiben beispielsweise in Froehler, J.Am.Chem.Soc. 114 (1992) 8320) und/oder öffenkettige Zuckeranaloga (beschrieben beispielsweise in Vandendriessche et al., Tetrahedron 49 (1993) 7223) und/oder Bicyclo-Zuckeranaloga (beschrieben beispielsweise in M. Tarkov et al., Helv. Chim. Acta 76 (1993) 481). Die 2'-modifizierten Oligonucleotid-Analoga sind in Manoharan, Biochim. Biophys. Acta (1999) 117 und konformativ eingeschränkte Oligonucleotid-Analoga sind in Herdewijn, Biochim. Biopyhs. Acta (1999) 167 detailliert beschrieben.
e) die Modifikation beziehungsweise den vollständigen oder teilweisen Ersatz der natürlichen Nucleosid-Basen, beispielsweise durch 5-(Hydroxymethyl)uracil, 5-Aminouracil, Pseudouracil, Pseudoisocytosin, Dihydrouracil, 5-(C₁-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin oder 7-Deaza-7-substituierte Purine beinhalten. Heterozyklische Basenmodifikationen sind beispielsweise in Herdewijn, Antisense & Nucl. Acid Drug Dev. (2000) 297 beschrieben.

Die chemische Modifikation des Oligonucleotids umfaßt weiterhin die Konjugation eines Oligonucleotids mit einem oder mehreren Molekülen, welche die Eigenschaften (beispielsweise Nucleasestablilität, Affinität zur Target-Sequenz, Pharmakokinetik) des Oligonucleotids günstig beeinflussen und/oder bei der Hybridisierung des modifizierten Oligonucleotids an die Target-Sequenz diese unter Bindung und/oder Quervernetzung angreift (Oligonucleotid-Konjugate). Beispiele dafür sind Konjugate mit Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie (C₁₂-C₂₀)-Alkyl, mit Lipiden wie 1,2-Di-hexadecyl-rac-glycerin, mit Steroiden wie Cholesterin oder Testosteron, mit Vitaminen wie Vitamin E, mit Poly- bzw. Oligo-ethylengylcol, mit (C₁₂-C₁₈)-Alkyl-Phosphatdiestern und/oder mit -O-CH₂-CH(OH)-O-(C₁₂-C₁₈)-Alkyl. Solche Moleküle können am 5'- und/oder am 3'-Ende und/oder innerhalb der Sequenz, z.B. an eine Nucleobase konjugiert sein. Dem Fachmann bekannte Oligonucleotid-Konjugate sind beispielsweise in Manoharan (2001) Conjugated Oligonucleotides in Antisense technology. In: Crooke (Herausgeber) Antisense Technology. Marcel Dekker, Nwe York, beschrieben.

Eine spezielle Ausführungsform der chemischen Modifikation betrifft die Konjugation des Oligonucleotids a) mit lipophilen Molekülen, beispielsweise (C₁₂-C₂₀)-Alkyl, b) mit Steroiden wie Cholesterin und/oder Testosteron, c) mit Poly- und/oder Oligoethylenglykol, d) mit Vitamin E, e) mit Interkalatoren wie Pyren, f) mit (C₁₄-C₁₈)-Alkyl-Phosphatdiestern und/oder g) mit -O-CH₂-CH(OH)-O-(C₁₂-C₁₆)-Alkyl.

Eine weitere spezielle Ausführungsform der chemischen Modifikation betrifft die Derivatisierung des Oligonucleotids wie in HMR 99/L045 beschrieben als Arylester-Konjugat, beispielsweise als FDA-Konjugat, wodurch die zelluläre Aufnahme der Oligonucleotide begünstigt wird.

Verfahren zur Herstellung der Oligonucleotid-Derivate sind dem Fachmann bekannt und z.B. in Uhlmann, E. & Peyman, A., Chem. Rev. 90 (1990) 543 und/oder M. Manoharan in "Antisense Research and Applications", Crooke and Lebleu, Eds., CRC Press, Boca Raton, 1993, Chapter 17, S.303ff. und/oder EP-A 0 552 766 beschrieben.

In weiteren speziellen Ausführungsformen der Erfindung kann das Oligonucleotid am 5'-Ende eine 5'-5'-Inversionen aufweisen. Diese Art der chemischen Modifikation ist dem Fachmann bekannt und beispielsweise in M. Koga et al., J. Org. Chem. 56 (1991) 3757 beschrieben. Das 5'-Ende ist zudem eine bevorzugte Position zur Konjugation des Oligonucleotids mit einem oder mehreren Molekülen, welche die Eigenschaften (beispielsweise Nucleasestablilität, zelluläre Aufnahme, Affinität zur Target-Sequenz, Pharmakokinetik) des Oligonucleotids günstig beeinflussen.

Die Erfindung betrifft ferner Verfahren zur Herstellung der Oligonucleotide. Die beschriebenen Oligonucleotide können mit Hilfe verschiedener bekannter, chemischer Verfahren, z.B. wie in Eckstein, F. (1991) "Oligonucleotides and Analogues, A Practical Approach", IRL Press, Oxford beschrieben, hergestellt werden. Die Oligonucleotide können auch durch Verfahren hergestellt werden, die gegebenenfalls einen oder mehrere enzymatische Schritte enthalten.

Die Erfindung betrifft weiterhin die Verwendung der Oligonucleotide zur Modulation sowie zur ganzen oder teilweisen Inhibition der Expression von bestimmten Target-Genen, beispielsweise zur ganzen oder teilweisen Inhibition der Translation. Die Erfindung betrifft weiterhin die Verwendung der Oligonucleotide zur Modulation sowie zur ganzen oder teilweisen Inhibition der Expression in Zellen, die nur wenig, keine oder defekte 2'5'-Oligoadenylat-Synthase aufweisen.

Die Erfindung betrifft weiterhin die Verwendung der Oligonucleotide als Arzneimittel bzw. die Verwendung der Oligonucleotide zur Herstellung von Arzneimitteln. Insbesondere können die Oligonucleotide in Arzneimitteln, die zur Prävention und/oder Behandlung von Krankheiten, die mit der Expression bzw. einer Überexpression bestimmter Gene einhergehen, eingesetzt werden.

Die Erfindung betrifft weiterhin die Verwendung der Oligonucleotide bzw. von Arzneimitteln, die diese Oligonucleotide enthalten, zur Behandlung von Krankheiten, bei denen definierte Gene durch Überexpression ursächlich bzw. beteiligt ist.

Die Arzneimittel der vorliegenden Erfindung können beispielsweise zur Behandlung von Erkrankungen, die durch Viren hervorgerufen werden, beispielsweise durch CMV, HIV, HSV-1, HSV-2, Hepatitis B, Hepatitis C, oder Papilloma Viren, verwendet werden. Arzneimittel dieser Erfindung eignen sich besonders zur Behandlung von RNA Viren wie etwa Polioviren, VSV oder Influenza, insbesondere auch von doppelsträngigen RNA-Viren wie etwa Reoviren.

Die Arzneimittel der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für Krebsentstehung bzw. Krebswachstum verantwortlich sind. Solche Targets sind beispielsweise:
1) Nucleare Onkoproteine wie beispielsweise c-myc, N-myc, c-myb, c-fos, c-fos/jun, PCNA, p120
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl, c-ets
3) Zelluläre Rezeptoren wie beispielsweise EGF-Rezeptor, Her-2, c-erbA, VEGF-Rezeptor (KDR-1), Retinoid-Rezeptoren, Protein-Kinase regulatorische Untereinheit, c-fms, Tie-2, c-raf-1 Kinase, PKC-alpha, Protein Kinase A (R1 alpha)
4) Cytokine, Wachstumsfaktoren, Extrazelluläre Matrix wie beispielsweise CSF-1, IL- - 6, IL-1a, IL-1b, IL-2, IL-4, IL-6, IL-8, bFGF, VEGF, Myeloblastin, Fibronectin.
5) Inhibitoren von Tumor Suppressor Genen wie beispielsweise MDM-2.

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Die Arzneimittel der vorliegenden Erfindung eignen sich beispielsweise auch zur Verhinderung der Restenose. Beispielsweise können dabei Oligonucleotid-Sequenzen zum Einsatz kommen, die gegen Targets gerichtet sind, die für Proliferation oder Migration verantwortlich sind. Solche Targets sind beispielsweise:
1) Nucleare Transaktivator-Proteine und Cycline wie beispielsweise c-myc, c-myb, c-fos, c-fos/jun, Cycline und cdc2-Kinase
2) Mitogene oder Wachstumsfaktoren wie beispielsweise PDGF, bFGF, VEGF, EGF, HB-EGF und TGF-β.
3) Zelluläre Rezeptoren wie beispielsweise bFGF-Rezeptor, EGF-Rezeptor und PDGF-Rezeptor.

Die Erfindung betrifft ferner Oligonucleotide zur Behandlung von Asthma, wobei die Expression des Adenosin-A1-Rezeptors, Adenosin-A3-Rezeptors, Bradikinin-Rezeptors oder von IL-13 mit Hilfe geeigneter Oligonucleotide inhibiert werden.

Die Erfindung betrifft beispielsweise auch Oligonucleotide zur Behandlung von Herz-Kreislauf-Krankheiten, wobei zum Beispiel die Expression des β1-adrenergen Rezeptors oder eines Proteins aus der EDG-Familie, wie beispielsweise Edg-1, gehemmt wird.

Die Erfindung betrifft beispielsweise auch Oligonucleotide zur Behandlung von Diabetes, wobei zum Beispiel die Expression von PTP-1 B gehemmt wird.

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Bevorzugte Verabreichungsformen sind topische Applikationen, lokale Applikationen wie beispielsweise mit Hilfe eines Katheters oder durch Inhalation, Injektionen bzw. Infusionen und die orale Verabreichung. Zur Injektion werden die Oligonucleotid-Derivate in einer flüssigen Lösung, vorzugsweise in einem physiologisch annehmbaren Puffer, wie z.B. Hank's Lösung oder Ringer's Lösung, formuliert. Die Oligonucleotide können aber auch in fester Form formuliert werden und vor dem Gebrauch gelöst oder suspendiert werden. Die für die systematische Verabreichungbevorzugten Dosierungen betragen ca. 0,01 mg/kg bis ca. 50 mg/kg Körpergewicht und Tag.

Die Erfindung betrifft weiterhin pharmazeutische Zubereitungen, die Oligonucleotide und/oder deren physiologisch verträgliche Salze neben pharmazeutisch einwandfreien Träger-und/oder Zusatzstoffen enthalten.

Die Oligonucleotide und/oder deren physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine topische, perkutane, parenterale oder enterale Anwendung gestatten und die als aktiven Bestandteil eine wirksame Dosis mindestens eines Oligonucleotids, neben üblichen pharmazeutisch einwandfreien Träger- und Zusatzstoffen enthalten. Die Zubereitungen enthalten normalerweise etwa 0,1 bis 90 Gew.-% der therapeutisch wirksamen Verbindung. Zur Behandlung von Hautkrankheiten wie beispielsweise Psoriasis oder Vitiligo wird eine topische Anwendung, z.B. in Form von Salben, Lotionen oder Tinkturen, Emulsionen, Suspensionen bevorzugt.Die Herstellung der pharmazeutischen Präparate erfolgt in an sich bekannter Weise, (z. B. Remingtons Pharmaceutical Sciences, Mack Publ. Co., Easton, PA.), wobei pharmazeutisch inerte anorganische und/oder organische Trägerstoffe verwendet werden. Für die Herstellung von Pillen, Tabletten, Dragees und Hartgelatinekapseln kann man z.B. Lactose, Maisstärke und/oder Derivate derselben, Talk, Stearinsäure und/oder deren Salze etc. verwenden. Trägerstoffe für Weichgelatinekapseln und/oder Suppositorien sind z.B. Fette, Wachse, halbfeste und flüssige Polyole, natürliche und/oder gehärtete Öle etc. Als Trägerstoffe für die Herstellung von Lösungen und/oder Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate und/oder Rods eignen sich Mischpolymerisate aus Glykolsäure und Milchsäure. Darüber hinaus sind Liposomenformulierungen, die dem Fachmann bekannt sind, geeignet (N. Weiner, Drug Develop Ind Pharm 15 (1989) 1523; "Liposome Dermatics, Springer Verlag 1992), beispielsweise HVJ-Liposomen (Hayashi, Gene Therapy 3 (1996) 878). Die dermale Applikation kann beispielsweise auch auch unter Zuhilfenahme ionophoretischer oder Methoden und/oder mit Hilfe der Elektroporation erfolgen. Darüber hinaus können Lipofectine und andere Carriersysteme, beispielsweise solche, die in der Gentherapie Anwendung finden, verwendet werden. Insbesondere sind Systeme geeignet, mit deren Hilfe Oligonucleotide mit großer Effizienz in eukaryotische Zellen eingebracht werden können.

Eine pharmazeutische Zubereitung kann neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel und/oder Lösungsvermittler und/oder Mittel zur Erzielung eines Depoteffekts, sowie Salze zur Veränderung des osmotischen Drucks, Überzugsmittel und/oder Antioxidantien enthalten. Sie können auch zwei oder mehrere verschiedene Oligonucleotide und/oder deren physiologisch verträgliche Salze enthalten sowie ferner neben mindestens einem Oligonucleotid einen oder mehrere andere therapeutisch wirksame Stoffe.

Die Dosis kann innerhalb weiter Grenzen variieren und ist in jedem einzelnen Fall den individuellen Gegebenheiten anzupassen.

### Beispiele

### 1. Synthese der Oligonucleotide der Formel 1

### a) 3' aaaaaaCUUCGCUUCCAACACCUAGAC

(Die mit kleinen Buchstaben gekennzeichneten Basen weisen eine 2'5'-Internucleosid-Bindung auf).

Die Synthesen wurden am ABI 394 DNA oder Expedite Synthesizer (Fa. Applied Biosystems, Weiterstadt) durchgeführt. Es wurden die vom Hersteller empfohlenen Synthesezyklen verwendet, wobei aber für die Ribonucleosid-2'-O-Phosphoramidite die Kondensation verdoppelt wurde (mit je 400 sec Kupplungszeit) und die Jodoxidation auf 30 sec verlängert wurde. Als Festphase wurde ein 1000 Å Controlled Pore Glass (CPG)-Träger eingesetzt, der 5'-O-Dimethoxytrityl-N-6-benzoyladenosin (NSS-6101-10A, Chemgenes, Waltham, MA) über die 2' bzw 3'-Position des Zuckers gebunden hielt. Nach Abspaltung der 5'-O-Dimethoxytrityl-Gruppe mit Trichloressigsäure wurde der 2'5'-verknüpfte Oligonucleotidteil durch fünfmalige Kondensation mit 5'-O-Dimethoxytrityl-N-6-benzoyl-3'-O-tert. Butyldimethylsilyl-adenosin-2'-O-Phosphoramidit (ANP-5681, Chemgenes) aufgebaut. Danach wurde der 3'5'-verknüpfte Oligonucleotidteil durch wiederholte Kondensation mit den entsprechenden 5'-O-Dimethoxytrityl-2'-O-tert. Butyldimethylsilyl-nucleosid-3'-O-Phosphoramiditen (ANP-5671 bis ANP-5680, Chemgenes) aufgebaut. Zur Abspaltung des Oligomers vom Träger und Entschützung der Phosphat- und Aminoschutzgruppen wurde der CPG-Träger mit 750 µL conc. Ammoniak/Ethanol (3:1, v:v) für 24 Stunden bei 30°C geschüttelt. Der Überstand wurde vom Träger getrennt und dieser nochmals zweimal mit 150 µL conc. Ammoniak/Ethanol (3:1, v:v) gewaschen. Die vereinigten Überstände wurden im Vakuum konzentriert und der Rückstand in 1200 µL Triethylaminx3HF (sehr giftig) zur Abspaltung der Silyl-Schutzgruppen für 24 Stunden bei 30°C geschüttelt. Dann gibt man 700 µL n-Butanol zu, kühlt das Gemisch für 30 Minuten auf Trockeneis und zentrifugiert. Das Pellet wurde noch zweimal mit Butanol gewaschen. Anschließend wurde noch eine Kochsalzfällung durchgeführt. Man erhielt 112 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8527.2, gef. 8527.5).

### b) 3' a*a*a a-C*U*U*C G C*U*U C*C A A*C A C*C*U A G A*C

Die Synthese wurde analog zu Beispiel 1a durchgeführt, wobei der 2'5'-verknüpfte Oligonucleotidteil durch dreimalige Kondensation mit 5'-O-Dimethoxytrityl-N-6-benzoyl-3'-O-tert. Butyldimethylsilyl-adenosin-2'-O-Phosphoramidit (ANP-5681, Chemgenes) aufgebaut wurde. Zur Einführung des Phosphorothioat-Restes wurde im jeweiligen Oxidationsschritt anstelle der Jodlösung das Beaucage-Reagenz (RN-1535, Chemgenes, Waltham, MA) eingesetzt. Man erhielt 128 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8061.6, gef. 8062.8).

### 2. Inhibition der Luciferase-Expression in SL-3 Zellen

Zur Prüfung auf biologische Aktivität wurden folgende Oligonucleotide wie in Beispiel 1 beschrieben hergestellt und auf Inhibition der Luciferase-Aktivität geprüft.
a) 3' aaaaaaCUUCGCUUCCAACACCUAGAC
b) 3' a*a*a a-C*U*U*C G C*U*U C*C A A*C A C*C*U A G A*C

Transfektion: Am Vortag des Experiments wurden 2x10⁶ Zellen/mL in 6-Well Platten ausplattiert. Die Oligonucleotide wurden in 100µL SF 900II SFM (SF-900 Serumfreies Insektenmedium II; Gibco BRL 10902-096) aufgenommen. Zur Transfektion wurden 10µL Lipofectin (1 mg/mL; Gibco BRL) mit 100µL SF 900II SFM gemischt und 15 min bei Raumtemperatur inkubiert. Danach wurden der Lipofectin-Mix und die Nucleinsäure zusammen pipettiert und 15-45 min bei Raumtemperatur inkubiert. In der Zwischenzeit wurden die Zellen mit 3ml serumfreien Medium gewaschen, und nacheinander mit 800µL SF 90011 SFM und der Nucleinsäure/Lipofectin-Mischung versetzt und über Nacht bei 25 Grade inkubiert. Am nächsten Tag gibt man 1 mL Medium und Serum (Gibco BRL 10122-166; Endkonzentration 2%) zu.

Dual-Luciferase Reporter (DLR; Promega E1960) Assay System:
(http://www.promega.com/catalog)

Der DLR Assay von Promega erlaubt die sequenzielle Bestimmung der Aktivität der Firefly Luciferase und Renilla Luciferase, die sich in ihrer Nucleinsäuresequenz unterscheiden, aus einer einzigen Probe. Die zu messenden Oligonucleotide gemäß Formel I waren gegen die Firefly Luciferase gerichtet. Es sollte also nur die Firefly Luciferase, aber nicht die Renilla Luciferase Aktivität gehemmt werden. Neben der Hemmwirkung kann also auch auf Spezifität geprüft werden.

Zur passiven Lyse der Zellen in den Well- Plates wurde zunächst das Medium entfernt und die Zellen mit PBS (Phosphatgepufferte Kochsalzlösung (Gibco BRL 14200-067) gewaschen. Das Medium wurde komplett abgesaugt und anschließend der PLB (Passiver Lysis Puffer, 1:5 mit Wasser verdünnt; 500µL PLB (1x) in ein Well einer 6-Well Platte geben) dazu gegeben. Danach wurde 15 Minuten unter Schütteln bei Raumtemperatur inkubiert.

Zur Präparation des Luciferase Assay Reagenz II ( LARII) wurde das Luciferase Assay Substrat (LAS) in 10 mL des Luciferase Assay Puffers II (LAB II) resuspendiert. Zur Präparation des Stop & Glo Reagenz wurden 200µL des Stop & Glo Substrat (Lösung) in das Fläschen, welches trockenes Stop & Glo Substrat enthielt, gegeben und 10 Sekunden mit dem Vortexer durchmischt. Um eine 1x Stop& Glo Lösung zu bekommen, addiert man 20µL des 50x Stop & Glo Substrat und 1 mL Stop & Glo Puffer. Dies ist ausreichend für 10 Assays.

DLR-Assay: 100µL LAR 11 wurden zusammen mit 20µL Zell Lysat in ein Well gegeben und durch Auf- und Abpipettieren für 2-3 Sekunden gemischt. Nach der luminometrischen Messung der Firefly-Luciferaseaktivität wurden 100µL Stop & Glo Reagenz zugegeben, gemischt und dann die Renilla-Luciferase-Aktivität gemessen. Zur Bestimmung der Lumineszenz wurde das Luminometer Fluoroskan Ascent FL (Fa. Thermo Labsystems, Frankfurt) verwendet.

| Oligonucleotid | % Inhibition* |
|---|---|
| a) 3' aaaaaaCUUCGCUUCCAACACCUAGAC (RNA in antisense Orientierung, mit 2'5'-A) | 43 |
| b) 3' a*a*a a-C*U*U*C G C*U*U C*C A A*C A C*C*U A G A*C (RNA in antisense Orientierung, mit 2'5'-A) | 43 |
| c) 3' aaaaTTTTTTACCTTGTTGAAATGG (nicht komplementär zur Target RNA; sense Orientierung) | 12 |
| d) 3' a*a*a a-C*U*U*C G C*U*U C*C A A*C A C*C*U A G A*C (antisense Orientierung, unterstrichen 2'-O-mehtyl) | 7 |
| 5'-G A A G*C G A A G G*U*U G*U G G A U*C*U*G-teg (Seq ID Nr. 20; sense Orientierung, ohne 2'5'-A, teg : Triethylengycolphosphat) | 0 |
| 3'-teg-G*C*T*T C*C*A A*C A*C*C*T A G A*C*C*T*A (Seq ID Nr. 21; antisense Orientierung, DNA, unterstrichen 2'-O-mehtyl) | 0 |
| 1100 bp dsRNA | 94 |
| Ohne dsRNA | 0 |

| | |
|---|---|
| * % Inhibition der Firefly-Luciferase-Aktivitiät () | |

Das zur Firefly Luciferase komplementäre Oligonucleotid a) hemmte die Firefly-Luciferase-Aktivität wesentlich stärker als das nicht komplementäre Oligonucleotid c). Die Stabilisierung des Oligonucleotids mit Phosphorothioat-Resten (Oligonucleotid b) an bestimmten Positionen des Oligomers führte zu einer signifikant besseren Wirkung. Wenn die gesamte 3'5'-verknüpfte komplementäre Sequenz als 2'-O-Methyl-Derivat derivatisiert wurde, war nahezu keine Aktivität feststtellbar (Oligonucleotid d).

### 3. Inhibition der edg-1 Expression in primären menschlichen Nabelschnur-Zellen (HUVEC).

Zur Testung der erfindungsgemässen Oligonucleotide in primären menschlichen Zellen auf Inhibition der Genexpression, wurden diese auch gegen ein menschliches Gen bzw. die zugehörige RNA gerichtet und an menschlichen Zellen (HUVEC, human umbilical vein endothelial cells) getestet.
Die entsprechenden Oligonucleotide wurden synthetisiert. Die ersten beiden Sequenzen sind komplementär zur edg-1 RNA, während das dritte Oligonucleotid Basenmisspaarungen aufweist.

Als Kontroll-Oligonucleotide wurden die komplimentären Sequenzen (sense Orientierung) ohne 2'5'-Oligoadenylat Rest verwendet.
wobei * Phosphorothioat; a*a*a a ein 2'5'-verknüpftes Adenylat (partiell mit * modifiziert), und teg ein Triethyleneglycolphosphat ist.

Die mit Phosphorothioat an bestimmten Positionen modifizierten Oligoribonucleotid-Analoga wurden wie folgt in primären menschlichen Zellen zur Inhibition der Genexpression von Edg-1 in menschlichen Zellen (HUVEC, human umbilical vein endothelial cells) eingesetzt.

Zellen (HUVECs) und Detektion der zellulären Aufnahme.
Transfektion: 24h vor der eigentlichen Transfektion wurden primäre HUVECs (2. Passage, isoliert nach Jaffe et al., 1973, J. Clin.Invest 52, pp.2745) in einer Dichte von 2,5 x 10⁵ Zellen/Well in Ratten Kollagen-1 (Biocoat, #354400, Becton Dickinson) beschichtete 6-Well Platten ausplattiert. Zur Transfektion wurden 6 µl Lipofectin (1 mg/ml; Gibco BRL, # 18292-011) mit 200 µl serumfreien Opti-MEM 1-Medium ( Gibco BRL, 31985-047) gemischt und 15 Minuten bei Raumtemp-eratur inkubiert. Parallel hierzu wurden 10 µM (→ Endkonzentration 0,1 µM) bzw. eine 100 µM (→ Endkonzentration 1 µM) Oligonucleotid-lösung (in PBS, pH 7,4) im Verhältnis 1:10 mit serumfreien Opti-MEM 1-Medium verdünnt und im selben Volumen mit der präinkubierten Lipofectinlösung gemischt. Nach 15 minütiger Inkubation bei Raumtemperatur wurde dieser Ansatz auf 2 ml mit serumfreien Opti-MEM 1-Medium aufgefüllt und der einmal mit PBS gewaschene Zellrasen für 4 Stunden bei 37°C, 5% CO₂ und 95% Luftfeuchtigkeit mit diesem Ansatz inkubiert. Anschließend wurde der erneut mit PBS gewaschene Zellrasen mit Serum-haltigen EGM-Medium (CellSystems, # CC-3024 + EGM Zusätze # CC-3124) überschichtet und für weitere 24 bzw. 48 h inkubiert. Im Falle der Aufnahmestudien mit fluoreszenzmarkierten Oligonucleotiden wurden die Zellen nach 4 stündiger Inkubation mit 5% Paraformaldehyd (in PBS, pH 7.4) fixiert und direkt mit einem inversen Fluoreszenzmikroskop (Zeiss Axiovert 135M) durch Anregung mit einem FITC-Filter (Excitation: 490 nm, Emission: 510 nm) in einer 200-fachen Vergrößerung mit einer gekühlten CCD-Camera (ORCA-1, Bfi optilas) aufgenommen und durch die AQM2000-Software (Kinetic Imaging) prozessiert.
Western Blot Analyse: Zur Lyse der Zellen wurde der Zellrasen einmal mit PBS gewaschen und anschließend mit 200 µl/well 2 x Laemmli-Puffer (Bio-Rad #161-0737) überschichtet. Nach fünfminütiger Inkubation bei Raumtemperatur wurde das Zelllysat mit einem Zellschaber (Becton Dickinson, #3085) ge-sammelt und vor der diskontinuierlichen 12%-SDS-Polyacrylamidgelelektro-phorese (SDS-PAGE, Laemmli et al., 1970, Bio-Rad-Criterion-System #345-0014) für 5 Minuten bei 95°C gekocht und hiervon 45 µl pro Tasche aufgetragen. Der Gellauf erfolgte in 1 x Tris/Glycine/SDS Puffer (Bio-Rad # 161-0732). Für den Immunoblot wurde das Gel mit Hilfe der Bio-Rad Criterion Westernblot Apparatur (#170-4070) auf eine Nitrocellulose- (NC) Membran (Amersham # RPN 2020D) in 1 x Tris/Glycine Puffer (Bio-Rad #161-0732, + 10% Methanol) transferiert. Anschließend wurde die NC-Membran für eine Stunde bei Raumtemperatur mit 1 x TBS-Puffer (Bio-Rad # 170-6435), der 5% Milchpulver ("Blotto", Bio-Rad #170-6404) und 0,1% Tween 20 (Bio-Rad # 170-6531) enthielt abgesättigt. Nach dreimaligem Waschen der Membran in Blotto-freiem TBS-Tween (TBST) Puffer wurde die Membran mit dem anti-hEDG-1 Primärantikörper (polyklonales Kaninchenserum, das durch Immunisierung mit der EDG-1-spezifischen Peptidsequenz CKAHRSSVSDYVNYD, gekoppelt an KLH, gewonnen und gegen die o.g. Peptidsequenz affinitätsgereinigt wurde) über Nacht bei 4°C in einer 1:50 Verdünnung in TBST-Blotto inkubiert. Nach dreimaligem Waschen mit TBST wurde der Sekundärantikörper (anti-Kaninchen, alkalische Phosphatase gekoppellt, Dianova # 111-055-045) in einer 1:2000 Verdünnung in TBST-Blotto für eine Stunde bei Raumtemperatur inkubiert. Nach erneutem Waschen (Siehe oben) erfolgte die ECF ("enhanced chemifluorescent")-Detektionsreaktion (Amersham #RPN5785), wobei die mit Plastikfolie abgedeckte NC-Membran mit 1 ml ECF-Substrat (Amersham Pharmacia #RPN5785) für 5 Minuten bei Raumtemperatur inkubiert und mit einem Fluor-Imager 595 Scanner (Amersham Pharmacia) detektiert wurde. Die Quantifizierung erfolgte mit der ImageQuant Software (Amersham Pharmacia), wobei gegen das β-Tubulinsignal normalisiert wurde, das nach einmaligem Entfärben (Alpha Diagnostic Kit # 90100) der NC-Membran und durch Inkubation mit dem β-Tubulin spezifischen Primärantikörper (affinitäts-gereinigter Kaninchen Antikörper, Santa Cruz # sc-9104) nach der oben beschriebenen Methode, erhalten wurde.

| | EDG-1 Protein (% der Kontrolle) | | | | | |
|---|---|---|---|---|---|---|
| Konzentr ation (µM) | Oligo #2 (Region "175") | Oligo #3 (Region "175") | Oligo #5 (Region "725") | Oligo #6 (Region "725") | Oligo #7 mismatch | Oligo #8 mismatch |
| 0 | 100,0 | 100,0 | 100,0 | 100,0 | 100,0 | 100,0 |
| 0,01 | 87,7 | 51,4 | 98,6 | 47,2 | 89,4 | 128,3 |
| 0,05 | 100,8 | 44,2 | 129,3 | 35,5 | 109,7 | 107,5 |
| 0,1 | 103,0 | 35,5 | 109,4 | 25,1 | 121,8 | 103,6 |
| 0,5 | 119,2 | 40,3 | 107,2 | 27,1 | 95,7 | 85,6 |
| 1,0 | 104,4 | 34,0 | 96,2 | 22,6 | 100,1 | 83,5 |

Die Behandlung der primären HUVEC-Zellen mit den erfindungsgemässen chemisch modifizierten einzelsträngigen Oligoribonucleotiden führte zu einer dosisabhängiggen Hemmung der Expression von edg-1. Nur die Oligoribonucleotide #3 und #6 mit der Antisense Orientierung hemmten die Genexpression, während die Oligoribonucleotide #2 und #5 mit der Sense Orientierung die Expression nicht hemmten. Die Hemmung erwies sich als Zielgen-spezifisch, da nur die Edg-1 Protein-Spiegel, aber nicht die Tubulin-Spiegel nach Behandlung mit den edg-1-spezifischen Oligoribonucleotide #3 und #6 abgesenkt waren. Die Hemmung erwies sich auch als sequenzspezifisch hinsichtlich der eingesetzten Oligoribonucleotide, da nur die zu edg-1 homologen Oligoribonucleotide #3 und #6 die Expression von edg-1 hemmten, während das Oligoribonucleotid #8 mit der Antisense-Orientierung, das sich in 5 Nucleotiden von der edg-1 Sequenz unterscheidet, die edg-1 Expression nicht hemmte.

## Patentansprüche

1. Oligonucleotid der Formel I
5'-(N)ₓ-(Z)ₙ Formel I
wobei
N natürlich oder nicht natürlich vorkommende Ribonucleotide sind, die zumindest teilweise zu einer Ziel-RNA komplementär sind,
x unabhängig gleich 10 bis 100,
n gleich 2 bis 20,
Z natürlich oder nicht natürlich vorkommende Nucleotide sind, die mit einer 2'5'-Internucleosid-Bindung verknüpft sind,
mit der Maßgabe, dass deren homologe Ziel-RNA eines der folgenden Sequenzmuster aufweist
5'-(U)ᵥ-(N)_{z}-(U)_{w}
5'-(U)ᵥ-(N)_{z}-UX
5'-UX-(N)_{z}-UX und
5'-(U)ᵥ-(N)_{z}
wobei v unabhängig gleich 2 bis 20 ist,
wobei w unabhängig gleich 2 bis 20 ist,
z unabhängig 15 bis 25 ist,
U gleich Uridin, N gleich Adenosin (A), Guanosin (G), Cytidin (C) oder U und X gleich A, G oder C, bevorzugt A, sind,
bzw. deren physiologisch verträglichen Salze.

2. Oligonucleotid der Formel I nach Anspruch 1, wobei x gleich 15 bis 45 ist.

3. Oligonucleotid der Formel I nach Anspruch 2, wobei x gleich 16 bis 25 ist.

4. Oligonucleotid der Formel I nach einem oder mehreren der Ansprüche 1 bis 3, wobei n gleich 2 bis 10 ist.

5. Oligonucleotid der Formel I nach Anspruch 4, wobei n gleich 3 bis 6 ist.

6. Oligonucleotid der Formel I nach einem oder mehreren der Ansprüche 1 bis 5, wobei v gleich 2 bis 10 ist.

7. Oligonucleotid der Formel I nach Anspruch 6, wobei v gleich 3 bis 6 ist.

8. Oligonucleotid der Formel I nach einem der Ansprüche 6 oder 7, wobei w gleich 2 bis 10 ist.

9. Oligonucleotid der Formel I nach Anspruch 8, wobei w gleich 3 bis 6 ist.

10. Oligonucleotid der Formel I nach einem oder mehreren der Ansprüche 1 bis 9, wobei z gleich 16 bis 23 ist.

11. Oligonucleotid der Formel I nach Anspruch 10, wobei z gleich 19 bis 21 ist.

12. Oligonucleotid der Formel I nach einem oder mehreren der Ansprüche 1 bis 11, wobei Z für Adenosin oder 3'-Deoxyadenosin steht.

13. Oligonucleotid der Formel I nach einem oder mehreren der Ansprüche 1 bis 12, in denen eine oder mehrere natürliche Phosphodiester-Brücken durch unnatürliche Internucleotid-Brücken, welche gegen Nuclease-Abbau stabilisieren, ersetzt sind.

14. Oligonucleotid der Formel I nach einem oder mehreren der Ansprüche 1 bis 13, in denen eine oder mehrere natürliche Phosphodiester-Bindungen durch Phosphorothioat-Bindungen ersetzt sind.

15. Oligonucleotid der Formel I nach einem oder mehreren der Ansprüche 1 bis 14, in denen mehrere natürliche Phosphodiester-Bindungen durch Phosphorothioat-Bindungen ersetzt sind, wobei sich diese Modifikationen an den Enden und internen Pyrimidin-Nucleotiden befinden.

16. Verfahren zur Hemmung der Genexpression eines Ziel-Gens in einer Zelle in vitro unter Zuhilfenahme eines oder mehrer Oligonucleotide nach einem oder mehreren der Ansprüche 1 bis 15, wobei zuerst ein zu einem entsprechenden Ziel-Gen komplementäres Oligonucleotid hergestellt wird, dieses Oligonucleotid in eine Zelle eingeführt wird, die Zelle inkubiert wird und anschließend die Hemmung der Genexpression des Ziel-Gens durch Vergleichsmessungen an der Menge der entsprechenden mRNA oder des entsprechenden Genprodukts in einer Kontrollzelle bestimmt wird.

17. In vitro-Verfahren nach Anspruch 16 zur Hemmung der Genexpression eines Ziel-Gens in einer Zelle, in der die 2'5'-Oligoadenylat-Synthase im Vergleich zu einer Kontrollzelle unterexprimiert oder defekt ist.

18. Arzneimittel enthaltend ein Oligonucleotid nach einem oder mehreren der Ansprüche 1 bis 15 sowie Zusatz- und/oder Trägerstoffen und gegebenenfalls Hilfsstoffe zur Herstellung oder Formulierung eines Arzneimittels.

19. Arzneimittel nach Anspruch 18 zur Verwendung in der Tumortherapie.

20. Arzneimittel nach Anspruch 18 zur Verwendung in der Therapie oder Prävention von Infektionskrankheiten.

21. Arzneimittel nach Anspruch 18 zur Verwendung in der Therapie oder Prävention viraler Erkrankungen.

22. Arzneimittel nach Anspruch 18 zur Verwendung in der Therapie von Entzündungs- oder Asthma-Erkrankungen.

23. Arzneimittel nach Anspruch 18 zur Verwendung in der Therapie von Herz-Kreislauf- oder Stoffwechsel-Erkrankungen.

24. Verwendung eines Oligonucleotids nach einem oder mehreren der Ansprüche 1 bis 15 zur Identifizierung oder Validierung neuer therapeutischer Ziel-Gene.

25. Verwendung eines Oligonucleotids nach einem oder mehreren der Ansprüche 1 bis 15 zur Identifizierung oder Validierung neuer Ziel-Gene in der Pflanzenschutz-Forschung.

26. Verfahren zur Herstellung eines Oligonucleotids nach einem oder mehreren der Ansprüche 1 bis 15, wobei die Oligonucleotide zuerst in Lösung oder an der Festphase durch sukzessive oder blockweise Kupplung hergestellt und nach der Herstellung isoliert und gereinigt werden.

27. Verfahren zur Herstellung eines Arzneimittels, wobei ein Oligonucleotid-Derivat gemäß Anspruch 26 hergestellt wird, und
gegebenenfalls mit weiteren Zusatz- und/oder Trägerstoffen und gegebenenfalls Hilfsstoffen versetzt wird.

## Claims

1. An oligonucleotide of the formula I
5'-(N)ₓ-(Z)ₙ Formula I
where
N are naturally or not naturally occurring ribonucleotides which are at least partly complementary to a target RNA,
x is independently 10 to 100,
n is 2 to 20,
Z are naturally or not naturally occurring nucleotides which are linked to a 2'5' internucleoside bond,
with the proviso that its homologous target RNA has one of the following sequence patterns
5'-(U)ᵥ-(N)_{z}-(U)_{w}
5'-(U)ᵥ-(N)_{z}-UX
5'-UX-(N)_{z}-UX and
5'-(U)ᵥ-(N)_{z},
where v is independently 2 to 20,
where w is independently 2 to 20,
z is independently 15 to 25,
U is uridine, N is adenosine (A), guanosine (G), cytidine (C) or U and X is A, G or C, preferably A
and to its physiologically tolerated salts.

2. The oligonucleotide of the formula I as claimed in claim 1, wherein x is 15 to 45.

3. The oligonucleotide of the formula I as claimed in claim 2, wherein x is 16 to 25.

4. The oligonucleotide of the formula I as claimed in one or more of claims 1 to 3, wherein n is 2 to 10.

5. The oligonucleotide of the formula I as claimed in claim 4, wherein n is 3 to 6.

6. The oligonucleotide of the formula I as claimed in one or more of claims 1 to 5, wherein v is 2 to 10.

7. The oligonucleotide of the formula I as claimed in claim 6, wherein v is 3 to 6.

8. The oligonucleotide of the formula I as claimed in one of claims 6 or 7, wherein w is 2 to 10.

9. The oligonucleotide of the formula I as claimed in claim 8, wherein w is 3 to 6.

10. The oligonucleotide of the formula I as claimed in one or more of claims 1 to 9, wherein z is 16 to 23.

11. The oligonucleotide of the formula I as claimed in claim 10, wherein z is 19 to 21.

12. The oligonucleotide of the formula I as claimed in one or more of claims 1 to 11, wherein Z is adenosine or 3'-deoxyadenosine.

13. The oligonucleotide of the formula I as claimed in one or more of claims 1 to 12, in which one or more natural phosphodiester bonds have been replaced by unnatural internucleotide bonds which stabilize against nuclease degradation.

14. The oligonucleotide of the formula I as claimed in one or more of claims 1 to 13, in which one or more natural phosphodiester bonds have been replaced by phosphorothioate bonds.

15. The oligonucleotide of the formula I as claimed in one or more of claims 1 to 14, in which a plurality of natural phosphodiester bonds have been replaced by phosphorothioate bonds, with said modifications being located on the ends and on internal pyrimidine nucleotides.

16. A method for inhibiting gene expression of a target gene in a cell in vitro with the aid of one or more oligonucleotides as claimed in one or more of claims 1 to 15, wherein first an oligonucleotide complementary to an appropriate target gene is prepared, said oligonucleotide is introduced into a cell, said cell is incubated and inhibition of the gene expression of the target gene is then determined by comparative measurements of the amount of the corresponding mRNA or corresponding gene product in a control cell.

17. The in vitro method as claimed in claim 16 for inhibiting gene expression of a target gene in a cell in which 2'5'-oligoadenylate synthase is underexpressed in comparison with a control cell or is defective.

18. A pharmaceutical comprising an oligonucleotide as claimed in one or more of claims 1 to 15 and also additives and/or carriers and, where appropriate, excipients for preparing or formulating a pharmaceutical.

19. The pharmaceutical as claimed in claim 18 for use in tumor therapy.

20. The pharmaceutical as claimed in claim 18 for use in the therapy or prevention of infectious diseases.

21. The pharmaceutical as claimed in claim 18 for use in the therapy or prevention of viral diseases.

22. The pharmaceutical as claimed in claim 18 for use in the therapy of inflammations or asthma.

23. The pharmaceutical as claimed in claim 18 for use in the therapy of cardiovascular or metabolic disorders.

24. The use of an oligonucleotide as claimed in one or more of claims 1 to 15 for identifying or validating novel therapeutic target genes.

25. The use of an oligonucleotide as claimed in one or more of claims 1 to 15 for identifying or validating novel target genes in crop protection research.

26. A method for preparing an oligonucleotide as claimed in one or more of claims 1 to 15, wherein the oligonucleotides are first prepared in solution or on the solid phase by successive coupling or coupling in blocks and are, after the preparation, isolated and purified.

27. A method for preparing a pharmaceutical, wherein an oligonucleotide derivative as claimed in claim 26 is prepared and, where appropriate, admixed with further additives and/or carriers and, where appropriate, excipients.

## Revendications

1. Oligonucléotide de formule I
5'-(N)ₓ-(Z)ₙ Formule 1
dans laquelle:
N représente des ribonucléotides naturels ou non naturels, qui sont au moins partiellement complémentaires d'un ARN cible,
x représente indépendamment une valeur de 10 à 100,
n représente une valeur de 2 à 20,
Z représente des nucléotides naturels ou non naturels, qui sont reliés par une liaison inter-nucléosidique de type 2'5',
à condition que leur ARN cible homologue présente l'un des profils de séquence suivants :
5'-(U)ᵥ-(N)_{z}-(U)_{w}
5'-(U)ᵥ-(N)_{z}-UX
5'-UX-(N)_{z}-UX et
5'-(U)ᵥ-(N)_{z}
dans lesquels :
v représente indépendamment une valeur de 2 à 20,
w représente indépendamment une valeur de 2 à 20,
z représente indépendamment une valeur de 15 à 25,
U représente l'uridine, N l'adénosine (A), la guanosine (G), la cytidine (C) ou U et X représentent A, G ou C, de préférence A,
ou ses sels acceptables au plan physiologique.

2. Oligonucléotide de formule I selon la revendication 1, dans lequel x représente une valeur de 15 à 45.

3. Oligonucléotide de formule I selon la revendication 2, dans lequel x représente une valeur de 16 à 25.

4. Oligonucléotide de formule I selon l'une ou plusieurs des revendications 1 à 3, dans lequel n représente une valeur de 2 à 10.

5. Oligonucléotide de formule I selon la revendication 4, dans lequel n représente une valeur de 3 à 6.

6. Oligonucléotide de formule I selon l'une ou plusieurs des revendications 1 à 5, dans lequel v représente une valeur de 2 à 10.

7. Oligonucléotide de formule I selon la revendication 6, dans lequel v représente une valeur de 3 à 6.

8. Oligonucléotide de formule I selon l'une des revendications 6 ou 7, dans lequel w représente une valeur de 2 à 10.

9. Oligonucléotide de formule I selon la revendication 8, dans lequel w représente une valeur de 3 à 6.

10. Oligonucléotide de formule I selon l'une ou plusieurs des revendications 1 à 9, dans lequel z représente une valeur de 16 à 23.

11. Oligonucléotide de formule I selon la revendication 10, dans lequel z représente une valeur de 19 à 21.

12. Oligonucléotide de formule I selon l'une ou plusieurs des revendications 1 à 11, dans lequel Z représente l'adénosine ou la 3'-désoxyadénosine.

13. Oligonucléotide de formule I selon l'une ou plusieurs des revendications 1 à 12, dans lequel un ou plusieurs ponts phosphodiester naturels sont remplacés par des ponts internucléotidiques non naturels, lesquels apportent un effet de stabilisation contre une dégradation par les nucléases.

14. Oligonucléotide de formule I selon l'une ou plusieurs des revendications 1 à 13, dans lequel on remplace une ou plusieurs liaisons phosphodiester naturelles par des liaisons phosphorothioate.

15. Oligonucléotide de formule I selon l'une ou plusieurs des revendications 1 à 14, dans lequel on remplace plusieurs liaisons phosphodiester naturelles par des liaisons phosphorothioate, ces modifications étant situées sur les extrémités et sur les nucléotides pyrimidiques internes.

16. Procédé pour inhiber l'expression génique d'un gène cible dans une cellule, *in vitro,* à l'aide d'un ou de plusieurs oligonucléotides selon l'une ou plusieurs des revendications 1 à 15, dans lequel on fabrique tout d'abord un oligonucléotide complémentaire à un gène cible correspondant, on introduit cet oligonucléotide dans une cellule, on incube la cellule, puis on détermine l'inhibition de l'expression génique du gène cible par des mesures comparatives sur la quantité d'ARNm correspondant ou du produit génique correspondant dans une cellule témoin.

17. Procédé *in vitro* selon la revendication 16, pour inhiber l'expression génique d'un gène cible dans une cellule, dans laquelle la 2'5'-oligoadénylate synthase est sous-exprimée ou déficiente en comparaison à une cellule témoin.

18. Produit pharmaceutique contenant un oligonucléotide selon l'une ou plusieurs des revendications 1 à 15, ainsi que des additifs et/ou des véhicules et, éventuellement, des adjuvants pour la fabrication ou la formulation d'un produit pharmaceutique.

19. Produit pharmaceutique selon la revendication 18, pour un usage en thérapie tumorale.

20. Produit pharmaceutique selon la revendication 18, pour un usage en thérapie ou en prévention de maladies infectieuses.

21. Produit pharmaceutique selon la revendication 18, pour un usage en thérapie ou en prévention de maladies virales.

22. Produit pharmaceutique selon la revendication 18, pour un usage en thérapie de maladies inflammatoires ou de maladies liées à l'asthme.

23. Produit pharmaceutique selon la revendication 18, pour un usage en thérapie de maladies cardiovasculaires ou métaboliques.

24. Utilisation d'un oligonucléotide selon l'une ou plusieurs des revendications 1 à 15, pour identifier ou valider de nouveaux gènes cibles thérapeutiques.

25. Utilisation d'un oligonucléotide selon l'une ou plusieurs des revendications 1 à 15, pour identifier ou valider de nouveaux gènes cibles en recherche phytosanitaire.

26. Procédé de fabrication d'un oligonucléotide selon l'une ou plusieurs des revendications 1 à 15, dans lequel les oligonucléotides sont tout d'abord fabriqués en solution ou en phase solide par des couplages successifs ou par couplage de séquences, puis sont isolés et purifiés après leur fabrication.

27. Procédé de fabrication d'un produit pharmaceutique, dans lequel on fabrique un dérivé oligonucléotidique selon la revendication 26, que l'on mélange, éventuellement, à d'autres additifs et/ou véhicules et, éventuellement, à d'autres adjuvants.
